# EUROPÄISCHE PATENTANMELDUNG

(11) **EP 0 882 435 A2**
(43) Veröffentlichungstag der Anmeldung: **09.12.1998**
(21) Anmeldenummer: 98108847.9
(22) Anmeldetag: 15.05.1998
(51) Int. Cl.: A61F 2/30, A61L 27/00

(54) **Implantat zum Einsatz bei Lebewesen, Verfahren zur Herstellung des Implantats sowie dessen Verwendung**

(30) Priorität: 04.06.1997 DE 19723287
(71) Anmelder: CASTOLIN S.A., CH-1025 Lausanne - St. Sulpice (CH)
(72) Erfinder: Wasserman, Christopher, 1135 Denens (CH); Lugscheider, Erich, Prof. Dr.-Techn., 52074 Aachen (DE); Nyland, A., Dr.-Ing., 52072 Aachen (DE); Steine, Hans-Theo, 1131 Tolochenaz (CH)
(74) Vertreter: Hiebsch, Gerhard F., Dipl.-Ing.

(57) **Zusammenfassung**

Bei einem Implantat aus metallischem Werkstoff mit thermisch aufgespritzter Beschichtung zum Einsatz bei Lebewesen, ist die Absorptionsgeschwindigkeit der Beschichtung zur Knochenbildung regelbar ausgebildet, und die Beschichtung weist zum einen am Übergangsbereich zum Knochen einen biologisch gut resorbierbaren Abschnitt sowie zum anderen am Übergang zum metallischen Werkstoff des Implantates eine Schicht aus einem biologisch stabilen Werkstoff auf. Auf dem metallischen Implantatteil soll eine Haftschicht angeordnet sein, bevorzugt eine Titanschicht.

## Beschreibung

Die Erfindung betrifft ein Implantat aus metallischem Werkstoff mit thermisch aufgespritzter Beschichtung zum Einsatz bei Lebewesen, ein Verfahren zur Herstellung des Implantats sowie dessen Verwendung.

Aus unterschiedlichen Werkstoffen -- wie nichtrostenden Stählen, Titan, Keramik od. dgl. -- hergestellte orthopädische und kieferchirurgische Implantate werden zuerst in situ angepaßt und anschließend mit einem Knochenzement eingekittet. Diese seit mehreren Jahrzehnten angewendete Vorgehensweise kann dadurch Probleme mit sich bringen, daß nach einer relativ kurzen Zeit Versprödungen des Knochenzements auftreten, welche zu einem Loslösen der Prothese führen und damit zu einem unerwünscht frühen Auswechseln derselben.

Im Zeitraum von 1970 bis 1980 wurden biokompatible Beschichtungen entwickelt, die teilweise vom Knochen resorbiert werden und die es erlauben, die Implantate ohne Einzementierung einzusetzen. Diese Methode hat den großen Vorteil, daß die Beschichtung des Implantats mit dem Knochen gut zusammenwächst und dadurch eine feste Verbindung zwischen Knochen und Implantat ausgebildet wird; Klinikaufenthalte der Patienten können hierdurch stark verkürzt werden, da die Belastung des Implantats schon nach etwa drei bis vier Tagen möglich ist. Als Nachteil wirkt sich allerdings aus, daß in manchen Fällen eine zu geringe Haftfestigkeit zwischen der Beschichtung und dem metallischen Implantat besteht, was dann einen Austausch kurz nach dem Einsetzen der Prothese bedingt.

Beim Ersetzen einer Prothese ist es notwendig, die an der Knochenoberfläche anhaftende Schicht der alten Prothese zu entfernen und den Knochen -- etwa zum Austausch eines Hüftgelenks -- nochmals durch einen Ausreibvorgang mit einem zu diesem Zweck entwickelten Werkzeug vorzubereiten. Da die aus Hydroxylapatit hergestellten Beschichtungen keramischen Charakter und hohe Härte aufweisen, ist jenes Ausreiben nur unter großen Anstrengungen mit einem höheren Werkzeugverschleiß und einem verhältnismäßig großen Zeitaufwand durchführbar. Ein weiterer Mangel besteht in dem hierbei auftretenden hohen Verlust an Knochensubstanz.

Auch Versuche mit anderen langzeitstabilen und resorbierbaren Beschichtungswerkstoffen aus natürlichem Perlmut oder aus Korallen, die in der Hauptsache aus Kalziumkarbonat bestehen und beispielhaft in WO 87 07 826, WO 94 17 838 und WO 94 26 322 beschrieben werden, ergaben nur teilweise brauchbare Ergebnisse. Da diese Beschichtungswerkstoffe wegen ihrer natürlichen Herkunft eine sehr schwankende Zusammensetzung aufweisen, konnten die daraus hergestellten Schichten lediglich in spezifischen Fällen in der Kieferchirurgie eingesetzt werden. Der EP-A-0 719 562 sowie der EP-A-0 230 570 ist die Verwendung von natürlichem Kalziumkarbonat und der EP-A-00 22 724 der Einsatz von syzithetisch hergestelltem Kalziumkarbonat zu entnehmen.

Beim Einsatz von Kalziumkarbonaten als Beschichtungswerkstoff wurde eine schnelle und gleichmäßige Resorption der Schicht -- in einer im Vergleich zu den anderen Beschichtungswerkstoffen kürzeren Zeit -- im Knochen festgestellt. Die Anwendung solcher Schichten ist daher sehr interessant, wenn durch die Form oder die Rauheit die Oberfläche der Prothese so ausgebildet ist, daß letztere durch die Neubildung des Knochens gehalten wird. Der Verbund zum metallischen Grundwerkstoff der Prothese zeigt allerdings in Hinsicht auf die erwähnten Hxdroxylapatite eine schlechtere Bindung.

In der US-A-4 542 539 wird eine gradierte Beschichtung beschrieben, bei der durch Leistungssteuerung des Plasmaspritzprozesses ein gradierter Porenanteil in Abhängigkeit von der Schichtstärke eingestellt wird.

In Kenntnis dieses Standes der Technik hat sich der Erfinder das Ziel gesetzt, die erkannten Nachteile zu beheben und eine verbesserte Beschichtung der Implantate zu schaffen.

Zur Lösung dieser Aufgabe führt die Lehre des unabhängigen Patentanspruches, die Unteransprüche geben günstige Weiterbildungen an.

Erfindungsgemäß ist bei dem Implantat aus metallischem Werkstoff die Absorptionsgeschwindigkeit der thermisch aufgespritzten Beschichtung zur Knochenbildung regelbar ausgebildet, zudem weist die Beschichtung zum einen am Übergangsbereich zum Knochen einen biologisch gut resorbierbaren Abschnitt und zum anderen am Übergang zum metallischen Werkstoff des Implantats eine Schicht aus einem biologisch stabilen Werkstoff auf.

Nach der Erkenntnis des Erfinders ist es möglich, eine biokompatible Beschichtung auf Wunsch so zu verbessern bzw. anzupassen, daß die Schicht in der jeweiligen Übergangszone die für diese notwendigen Eigenschaften aufweist. So resorbiert die Schicht in dem Übergang Knochen / Schicht schnell und soll im Übergang Implantat-Grundwerkstoff / Schicht biostabil bzw. schwer resorbierbar sein.

Das erfindungsgemäße Verfahren zeichnet sich dadurch aus, daß am Übergangsbereich zwischen Prothese bzw. Implantat und Knochen ein biologisch gut resorbierbarer Abschnitt sowie am Übergang zum metallischen Werkstoff des Implantates hin eine Schicht aus einem biologisch stabilen Werkstoff aufgetragen werden.

Nach einem weiteren Merkmal der Erfindung soll auf den metallischen Prothesenteil zur Verbesserung der Haftung eine Haftschicht aufgebracht werden, wobei sich als besonders günstig erwiesen hat, vor dem Auftragen der biokompatiblen Beschichtung eine Titanschicht zur Haftungsverbesserung anzuordnen.

Als günstig hat sich ein Beschichtungswerkstoff auf Kalium-, Kalzium- und/oder Natriumbasis erwiesen, vor allem auf Phosphat-, Oxid-, und/oder Karbonatbasis der Elemente Kalium, Kalzium und/oder Natrium.

Die oben erörterten Eigenschaften können durch das erfindungsgemäße Verfahren, bei dem ein oder mehrere Schichten verschiedene Beschichtungswerkstoffe so aufgebracht werden, daß an der Schichtoberfläche, also der dem Knochen zugekehrten Seite, eine schnell resorbierbare Schicht -- beispielsweise aus einem Kalziumkarbonat -- und an der Seite der Schicht zum Grundwerkstoff der metallischen Prothese beispielsweise eine Hydroxylapatitschicht aufweist. Die genaue Abstimmung der Eigenschaften kann in diesem Falle über die Anzahl und die Dicke der einzelnen wechselseitig aufgebrachten Schichten erfolgen.

Im Rahmen der Erfindung werden Beschichtungen aus zwei oder mehreren einzelnen Schichten verschiedener Zusammensetzung hergestellt. Dabei werden für die Beschichtung drei einzelne Schichten aus verschiedenen Verbindungen auf Kalziumbasis bevorzugt, etwa eine Schicht auf dem Implantat aus Hydroxylapatit, eine zur Knochenoberfläche weisende Schicht aus Kalziumkarbonat sowie eine Zwischenschicht eines Gemisches aus beiden Werkstoffen.

Vorteilhafterweise kann auch eine gradierte Schicht aufgebracht werden; die an der dem Knochen zugewandten Seite vorgesehene Schicht soll bevorzugt aus 100 Gew.-% Kalziumkarbonat oder ähnlichen schnell resorbierenden Stoffen bestehen, die dem Grundwerkstoff der Prothese zugewandte Schicht aus 100 Gew.-% biostabilem Stoff z. B. Hydroxylapatit.

Als Schichten für die Übergangszone zum Knochen können alle schneller resorbierbaren Stoffe verwendet werden, bei denen die Resorptionsgeschwindigkeit des aufgebrachten Biomaterials nicht höher liegt als die Wachstumsgeschwindigkeit des Knochens. Daher ist im Rahmen der Entwicklung eine Angleichung des Ausgangsmaterials an die Spritzschicht vorgegeben.

Für die Übergangszone an der Prothesenseite eignen sich die meisten Kalizium-Phosphatverbindungen, mit und ohne eine mehr oder weniger poröse Titanschicht zwischen Grundwerkstoff der Prothese und der biostabilen Schicht od. dgl. zur Verbesserung der Haftung. Erfindungsgemäß wird das Beschichtungssystem in einer APS-Plasmaanlage oder in einer LPPS-Plasmaanlage aufgebracht. Auch kann dazu eine Hochgeschwindigkeitflammspritzanlage (HVOF) -- gegebenenfalls eine solche mit Flüssigbrennsotff oder gasförmigem Brennstoff -- eingesetzt werden.

Außerdem hat es sich als günstig erwiesen, die verschiedenen Pulver über zwei getrennte Pulverfördersysteme der Flamme zuzuführen, wobei zwischen den beiden Pulverfördersystemen und der Spritzpistole -- zum Mischen der dosiert zugeführten Pulver -- eine Mischkammer angeordnet sein sollte.

Weitere Vorteile, Merkmale und Einzelheiten der Erfindung ergeben sich aus der nachfolgenden Beschreibung bevorzugter Ausführungsbeispiele.

### Beispiel 1

Ein Hüftgelenkschaft sollte zur Stabilisierung des Implantates an der Oberfläche mit einer biokompatiblen Schicht versehen werden, die auf der Knochenseite schnell resorbierbar und auf der Seite zur Prothese mit einer biostabilen bzw. nur sehr langsam resorbierbaren Schicht ausgestattet ist.

Als schnell resorbierbarer Schichtwerkstoff wurde ein Kalziumkarbonatpulver mit einem Anteil von etwa 45 Gew.-% Aragonit, Rest Calcit, verwendet. Zum Prothesengrundwerkstoff hin wurde als Kalzium-Phosphatpulver ein Hydroxylapatit verwendet.

Der Hüftgelenkschaft aus Titan wurde an den nichtzubeschichtenden Stellen mit einem Klebeband abgedeckt. Die beschichteten Stellen wurden anschließend mit Aluminiumoxydpulver einer Korngröße von 0,2 bis 0,5 mm durch Strahlen vorbereitet. Die Oberflächenrauheit lag nach dem Strahlen bei Ra = 10 bis 15 µm. Nach dem Strahlen wurde auf den Grundwerkstoff mit einer Plasmaspritzanlage -- die mit zwei Pulverfördereinrichtungen ausgerüstet und von denen eine mit Kalziumkarbonat sowie die andere mit Hydroxylapatite gefüllt war -- beschichtet.

Zuerst wurde die Oberfläche der Prothese aus dem einen Pulverförderer mit einer Hydroxylapatitschicht einer Schichtdicke von 50 bis 80 µm versehen und anschließend -- nach Umschalten auf den zweiten Pulverförderer -- eine zweite Lage aus Kalziumkarbonat mit einer Schichtstärke von 50 bis 70 µm aufgespritzt, so daß die Gesamtschichtdicke zwischen 100 bis 150 µm lag.

Nach dem Entfernen der Abdeckung und dem Reinigen der Oberfläche von Rückständen erfolgte die Endprüfung. Mit der so hergestellten Prothese wurden dann anschließend Proben für Versuche in vivo und in vitro entnommen.

### Beispiel 2

Dieser Vorgang zum Aufbringen einer Beschichtung auf einen Hüftgelenkschaft entsprach dem des Beispiels 1 mit dem Unterschied, daß zur Durchführung der Beschichtung eine Vakuumplasmaanlage verwendet wurde. Wegen der besseren Haftung des Schichtverbundes auf dem Grundwerkstoff der Prothese wurde vor dem eigentlichen Beschichtungsvorgang eine Titanschicht zur Verbesserung der Haftung aufgespritzt.

### Beispiel 3

Eine Hüftgelenkschale sollte auf der äußeren Seite in Richtung Hüftknochen mit einer Beschichtung versehen werden, die auf der Seite zum Knochen gut resorbierbar ist und auf der Seite der Hüftschale eine biostabile Schicht aufweist. Dazu wurde für die Knochenseite ein pulverförmiger Spritzwerkstoff auf Karbonatbasis und für die Prothesenseite einer auf Phosphatbasis ausgewählt.

Als Beschichtungsanlage wurde eine mit zwei Pulverfördereinheiten ausgerüstete Plasmaanlage verwendet. Zum Erzeugen der gradierten Beschichtung war am Ausgang der beiden Pulverfördereinheiten eine Mischkammer angeordnet, in der die aus den beiden Pulverförderer austretende Pulverströme vor der Zuleitung der Spritzdüse kontinuierlich gemischt werden, so daß der Spritzwerkstoff über die Ansteuerung der zwei Pulverförderer stufenlos über die gesamte Zusammensetzung verändert werden konnte.

Nach dem Vorbereiten der zu beschichtenden Prothesenoberfläche durch Abdecken der nicht zu beschichtenden Stellen mit einem Abdeckband und anschließendem Strahlen mit Korund auf eine Rauheit von 15 bis 20 µm wurde der Beschichtungsvorgang mit der Plasmaspritzpistole unter Verwendung eines Argon/Wasserstoffplasmas durchgeführt. Die Schichtdicke der fertigen gradierten Schicht lag bei 50 bis 180 µm.

Bei der nachträglich durchgeführten Kontrolle und der anschließenden Schliffprobe konnte eine einwandfreie Schichtqualität festgestellt werden.

### Beispiel 4

Das Beispiel 4 zum Herstellen einer Beschichtung auf einer Hüftgelenkschale entsprach dem Beispiel 3 mit dem Unterschied, daß zur Durchführung der Beschichtung eine Vakuumplasmaanlage verwendet wurde. Wegen der besseren Haftung des Schichtverbundes auf dem Grundwerkstoff der Prothese wurde vor dem eigentlichen Beschichtungsvorgang eine Titanschicht zur Verbesserung der Haftung aufgespritzt.

### Beispiel 5

An einer Knieprothese sollte eine aus mehreren Schichten mit verschiedenen Zusammensetzungen und unterschiedlichen Resorptionsverhalten bestehende Beschichtung aufgebracht werden. Als Werkstoff wurden dieselben mit der gleichen Schichtabstimmung wie in Beispiel 1 vorgesehen.

Zum Herstellen der Beschichtung wurde eine mit gasförmigem Brennstoff arbeitende Hochgeschwindigkeitsflammspritzanlage (HVOF Anlage) verwendet, die wie die in Beispiel 1 beschriebene Plasmaanlage mit zwei Pulverfördereinrichtungen ausgerüstet war.

Die Schichtdicke der vier übereinander aufgebrachten Schichten lag bei 20 µm und die der fertigen Schicht bei 80 µm.

Die anschließend durchgeführten Untersuchungen der aufgebrachten Schicht zeigte sehr gute Resultate.

### Beispiel 6

Das Beispiel 6 zum Herstellen einer Beschichtung auf einer Kniegelenkprothese entsprach dem Beispiel 5 mit dem Unterschied, daß zur Durchführung der Beschichtung eine Plasmaanlage verwendet wurde.

### Beispiel 7

Das Beispiel 7 zum Beschichten eines Hüftgelenkschaftes entspricht i.w. dem Beispiel 1 mit dem Unterschied, daß der Beschichtungsvorgang mit einer HVOF-Anlage durchgeführt wurde, die mit flüssigem Brennstoff arbeitet.

Die am Produkt durchgeführten Kontrollen ergaben hervorragende Werte.

## Patentansprüche

1. Implantat aus metallischem Werkstoff mit thermisch aufgespritzter Beschichtung zum Einsatz bei Lebewesen, bei dem die Absorptionsgeschwindigkeit der Beschichtung zur Knochenbildung regelbar ausgebildet ist sowie die Beschichtung zum einen am Übergangsbereich zum Knochen einen biologisch gut resorbierbaren Abschnitt aufweist und zum anderen am Übergang zum metallischen Werkstoff des Implantates eine Schicht aus einem biologisch stabilen Werkstoff.

2. Implantat nach Anspruch 1, gekennzeichnet durch eine Haftschicht auf dem metallischen Implantatteil, insbesondere durch eine Titanschicht als Haftschicht.

3. Implantat nach Anspruch 1 oder 2, gekennzeichnet durch einen Beschichtungswerkstoff auf Kalium-, Kalzium- und/oder Natriumbasis, insbesondere auf Phosphat-, Oxid-, und/oder Karbonatbasis der Elemente Kalium, Kalzium und/oder Natrium.

4. Implantat nach einem der Ansprüche 1 bis 3, gekennzeichnet durch einen Hauptanteil an Kalziumverbindungen im Beschichtungswerkstoff.

5. Implantat nach einem der Ansprüche 1 bis 4, gekennzeichnet durch eine Beschichtung aus zumindest zwei einzelnen Schichten verschiedener Verbindungen auf Kalziumbasis.

6. Implantat nach einem der Ansprüche 1 bis 5, gekennzeichnet durch eine auf der metallischen Implantatoberfläche vorgesehene Teilschicht aus einer Kalzimphosphatverbindung, wobei gegebenenfalls die auf der metallischen Implantatoberfläche vorhandene Teilschicht aus Kalzium-Hydroxylapatit besteht und/oder die mit dem Knochen in Kontakt stehende Seite der Teilschicht eine Kalziumkarbonatverbindung ist, insbesondere eine Kalziumkarbonatverbindung mit einer Calcit- oder Aragonit-Struktur.

7. Implantat nach einem der Ansprüche 1 bis 6, dadurch gekennzeichnet, daß die Beschichtung aus drei einzelnen Schichten verschiedener Verbindungen auf Kalziumbasis besteht, wobei gegebenenfalls eine Schicht auf dem Implantatteil aus Hydroxylapatit, eine zur Knochenoberfläche weisende Schicht aus Kalziumkarbonat und eine Zwischenschicht aus einem Gemisch der beiden Werkstoffe vorgesehen sind.

8. Verfahren zum Herstellen eines Implantats durch thermisches Spritzen nach wenigstens einem der Ansprüche 1 bis 7, bei dem am Übergangsbereich zum Knochen hin ein biologisch gut resorbierbarer Abschnitt sowie am Übergang zum metallischen Werkstoff des Implantats eine Schicht aus einem biologisch stabilen Werkstoff aufgetragen werden.

9. Verfahren nach Anspruch 8, dadurch gekennzeichnet, daß auf den metallischen Implantatteil eine Haftschicht aufgebracht wird, insbesondere vor dem Auftragen der biokompatiblen Beschichtung eine Titanschicht.

10. Verfahren nach Anspruch 8 oder 9, dadurch gekennzeichnet, daß ein Beschichtungswerksotff auf Kalium, Natrium- und/oder Kalziumbasis aufgetragen wird.

11. Verfahren nach einem der Ansprüche 8 bis 10, dadurch gekennzeichnet, daß ein Beschichtungswerkstoff auf Phosphat-, Oxid-, und/oder Karbonatbasis der Elemente Kalium, Kalzium und/oder Natrium aufgetragen wird.

12. Verfahren nach einem der Ansprüche 8 bis 11, gekennzeichnet durch einen Hauptanteil an Kalziumverbindungen im Beschichtungswerkstoff.

13. Verfahren nach einem der Ansprüche 8 bis 12, dadurch gekennzeichnet, daß die Beschichtung aus zumindest zwei einzelnen Schichten verschiedener Verbindungen auf Kalziumbasis aufgebaut wird.

14. Verfahren nach einem der Ansprüche 8 bis 13, dadurch gekennzeichnet, daß auf der metallischen Implantatoberfläche eine Teilschicht aus einer Kalziumphosphatverbindung aufgebaut, insbesondere die auf der metallischen Implantatoberfläche vorhandene Teilschicht aus Kalzium-Hydroxylapatit hergestellt, wird.

15. Verfahren nach Anspruch 14, dadurch gekennzeichnet, daß die mit dem Knochen in Kontakt stehende Seite der Teilschicht aus einer Kalziumkarbonatverbindung gefertigt wird, wobei gegebenenfalls die Kalziumkarbonatverbindung eine Calcit- oder Aragonit-Struktur aufweist.

16. Verfahren nach einem der Ansprüche 8 bis 15, dadurch gekennzeichnet, daß die Beschichtung aus drei einzelnen Schichten verschiedener Verbindungen auf Kalziumbasis hergestellt wird, wobei gegebenenfalls eine Schicht auf dem Implantatteil aus Hydroxylapatit, eine zur Knochenoberfläche weisende Schicht aus Kalziumkarbonat und eine Zwischenschicht aus einem Gemisch der beiden Werkstoffe hergestellt werden.

17. Verfahren nach einem der Ansprüche 8 bis 16, gekennzeichnet durch eine Beschichtung mit gradierter Abstufung von 0,1 bis 100 Gew.-% Hydroxylapatit, gegebenenfalls durch eine Beschichtung mit gradierter Abstufung von 100 bis 0,1 Gew.-% Kalziumkarbonatbasis.

18. Verfahren nach Anspruch 17, dadurch gekennzeichnet, daß die leicht resorbierbare Seite die Schicht mit 100 Gew-% Kalziumkarbonatbasis auf der Knochenkontaktseite und die Seite der Schicht mit 100 Gew.-% Hydroxylapatit auf der Seite zur metallischen Prothese hin angeordnet wird.

19. Verfahren nach einem der Ansprüche 8 bis 18, dadurch gekennzeichnet, daß das Beschichtungssystem in einer APS-Plasmaanlage oder in einer LPPS-Plasmaanlage aufgebracht wird.

20. Verfahren nach einem der Ansprüche 8 bis 18, dadurch gekennzeichnet, daß das Beschichtungssystem durch eine Hochgeschwindigkeitflammpritzanlage (HVOF) aufgetragen wird, gegebenenfalls eine solche Hochgeschwindigkeitflammspritzanlage mit Flüssigbrennstoff oder gasförmigem Brennstoff.

21. Verfahren nach wenigstens einem der Ansprüche 8 bis 20, dadurch gekennzeichnet, daß die verschiedenen Pulver über zwei getrennte Pulverfördersysteme zur Flamme zugeführt werden, gegebenenfalls durch eine zwischen den zwei Pulverfördereinrichtungen und der Spritzpistole zum Mischen der dosiert zugeführten Pulver angeordnete Mischkammer.

22. Verfahren nach wenigstens einem der Ansprüche 18 bis 20, dadurch gekennzeichnet, daß das Pulver aus zwei Fördersystemen über eine Zuleitung zum Brenner bzw. Spritzpistole zugeführt wird, gegebenenfalls durch eine zwischen den zwei Pulverfördereinrichtungen und der Spritzpistole zum Mischen der dosiert zugeführten Pulver angeordnete Mischkammer.

23. Verwendung des nach wenigstens einem der Ansprüche 18 bis 22 hergestellten beschichteten Implantates für orthopädische oder kieferchirurgisch implantierbare Prothesenteile.
